(19) Europäisches Patentamt
European Patent Office
Office européen des brevets

(11) Veröffentlichungsnummer: **0 309 392 B1**

(12) **EUROPÄISCHE PATENTSCHRIFT**

(45) Veröffentlichungstag der Patentschrift: **02.09.92**

(51) Int. Cl.$^5$: **C07C 309/29**

(21) Anmeldenummer: **88810522.8**

(22) Anmeldetag: **28.07.88**

(54) **Mono- und Dichlor-4,4'-diaminodiphenyl-5,5'-disulfonsäure.**

(30) Priorität: **07.08.87 GB 8718798**

(43) Veröffentlichungstag der Anmeldung:
**29.03.89 Patentblatt 89/13**

(45) Bekanntmachung des Hinweises auf die Patenterteilung:
**02.09.92 Patentblatt 92/36**

(84) Benannte Vertragsstaaten:
**DE FR GB IT**

(56) Entgegenhaltungen:
**DE-A- 2 260 907**
**DE-C- 47 426**
**FR-A- 690 112**
**FR-A- 1 486 851**
**US-A- 2 346 941**

(73) Patentinhaber: **CIBA-GEIGY AG**
**Klybeckstrasse 141**
**CH-4002 Basel(CH)**

(72) Erfinder: **Campbell, Colin Dennis, Dr.**
**1, Boghead Beith**
**Ayrshire Scotland KA15 1DZ(GB)**

EP 0 309 392 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue sulfonierte 3,3′-Dichlorbenzidine, ihre Herstellung und Verwendung.

In der deutschen Offenlegungsschrift Nr. 2 260 907 sind unsymmetrische Verbindungen der Formeln 1 oder 2

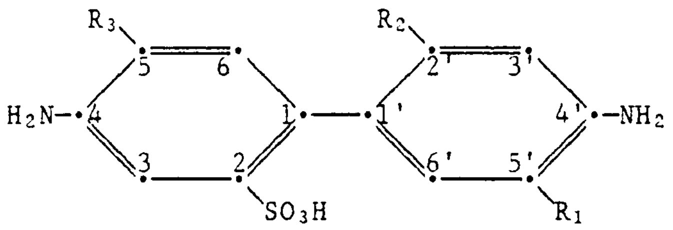

(1)

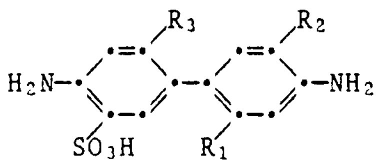

(2) beschrieben,

worin $R_1$, $R_2$ und $R_3$ -H, Halogen, $C_1$-$C_6$-Alkyl oder $C_1$-$C_6$-Alkoxy sind, ferner $R_2$ $-SO_3H$ bedeutet. Diese Verbindungen werden aus den entsprechenden Hydrazoverbindungen durch Benzidin-Umlagerung gewonnen. Bei den obigen Verbindungen der Formeln 1 oder 2 sind höchstens zwei der 3-, 3′-, 5- und 5′-Stellungen durch andere Substituenten als Wasserstoff besetzt.

Bestimmte neue sulfonierte 3,3′-Dichlorbenzidinderivate wurden nun hergestellt. Sie besitzen einen hohen Wert z.B. als Zwischenprodukte für Farbstoffzusätze in Pigmentzusammensetzungen und als Zwischenprodukte für Metall enthaltende Pigmente.

Die vorliegende Erfindung betrifft demnach Verbindungen der Formeln I oder II

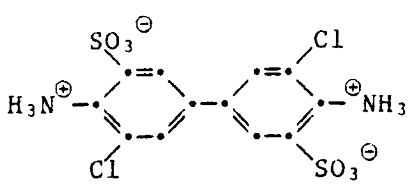 (I), 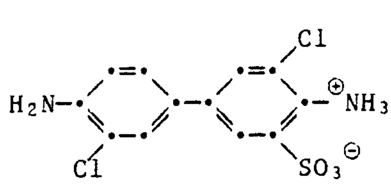 (II),

sowie deren Salze und Hydrate.

Die erfindungsgemässen Verbindungen sind als Zwitterionen dargestellt, wie sie bekanntlich im festen Zustand existieren. Die vorliegende Erfindung beschränkt sich jedoch nicht auf irgendwelche spezifischen ionischen Formen der neuen Verbindungen.

Der Wassergehalt in den Hydraten der obigen Verbindugen kann beachtlich variieren und hängt beispielsweise von der Trocknungstemperatur oder der Hygroskopizität dieser Verbindungen ab. Diese Hydrate können daher ein Mol Kirstall-Wasser oder mehr im Molekül der Verbindung der Formeln I und II, sowie deren Salze, haben.

Die anmeldungsgemässen Verbindungen können auch als Salze, die zwischen der (den) Sulfonsäure-Gruppe(n) und einer anorganischen oder organischen Base gebildet werden, isoliert werden. Beispiele derartiger Salze sind Metall-, Ammonium- oder Amin-Salze, insbesondere Alkalimetallsalze, Erdalkalimetallsalze, Metallsalze aus den Gruppen IIB oder VIIB des Periodensystems der Elemente, Ammoniumsalze oder Salze organischer Amine.

Spezifische Beispiele hierfür sind Lithium-, Natrium-, Kalium-, Calcium-, Zink- und Mangansalze, ferner Ammonium-, Triaethylamin- und Diaethylanilinsalze, sowie Tetraalkylammoniumsalze, wie z.B. Tetrabutylammoniumsalze.

Ein weiterer Erfindungsgegenstand betrifft ein Verfahren zur Herstellung der Verbindungen der Formeln I oder II, wonach man 3,3′-Dichlorbenzidinoder dessen Dichlorhydrat mit einer entsprechenden stöchiometrischen Menge Oleum bei erhöhter Temperatur reagieren lässt.

Das molare Verhältnis 3,3′-Dichlorbenzidin zu Oleum kann variieren und hängt davon ab, ob ein disulfoniertes Produkt gemäss Formel I oder eine monosulfonierte Verbindung gemäss Formel II hergestellt werden muss. Zur Gewinnung eines Produktes der Formel I beträgt das molare Verhältnis 3,3′-Dichlorbenzidin: Oleum vorzugsweise im wesentlichen 1:2, für Produkte der Formel II dagegen vorzugsweise in wesentlichen 1:1.

Falls erwünscht, kann man anstelle von 3,3′-Dichlorbenzidin 3,3′-Dichlorbenzidin-Dihydrochlorid verwenden, worin im vorliegenden Fall das Reaktionsgemisch, falls erwünscht, durch Verwendung konzentrierter Schwefelsäure als Lösungsmittel in Lösung gebracht werden kann.

Die Schwefeltrioxid-Konzentration im verwendeten Oleum liegt zweckmässig zwischen 20 und 30 Gewichtsprozent.

Die Reaktionstemperatur kann über einen breiten Bereich variieren, sie liegt aber zwischen 90°C und 180°C, vorzugsweise zwischen 100°C und 160°C.

Nach Vervollständigung der Reaktion kann das Reaktionsgemisch nach konventionellen Techniken aufgearbeitet werden.

Eine Alternativ-Methode zur Herstellung der Verbindungen der Formel I besteht darin, dass man eine Verbindung der Formel II mit Oleum, zweckmässig unter Verwendung der oben angegebenen Reaktionsbedingungen, reagieren lässt.

Die folgenden Beispiele illustrieren die vorliegende Erfindung.

<u>Beispiel 1:</u> <u>3,3′-Dichlor-4,4′-diaminodiphenyl-5,5′-disulfonsäure ausgehend von 3,3′-Dichlorbenzidin</u>

3,3′-Dichlorbenzidin als freie Base (50,6 g; 0,2 Mol) und als feines getrocknetes Pulver wird zu 25-30%igem Oleum (122 g; ca. 0,42 Mol $SO_3$) bei 20°C unter Rühren gegeben. Das heterogene Gemisch wird dann mit einem Oelbad erwärmt; dabei entsteht bei einer Temperatur von 100°C eine Lösung. Bei 130-140°C wird die Reaktionsmasse als dunkelgraues festes Produkt fest. Nach Abkühlung dieser Reaktionsmasse auf 80°C wird Eiswasser zugegeben, worauf das Produkt zu einer feinteiligen grauen Suspension vermahlen und anschliessend abfiltriert wird. Das Produkt ist etwas wasserlöslich und wird deshalb nicht gewaschen. Ausbeute: 82 g (= 95 % der Theorie).

<u>Beispiel 2:</u> <u>3,3′-Dichlor-4,4′-diaminodiphenyl-5,5′-disulfonsäure ausgehend von 3,3′-Dichlorbenzidin-Dichlorhydrat</u>

3,3′-Dichlorbenzidin-Dichlorhydrat wird zuerst bei 70°C während 16 Stunden getrocknet.

Zu 25-30 %igem Oleum (122 g; ca. 0,42 Mol $SO_3$) wird obiges getrocknetes 3,3′-Dichlorbenzidin-Dichlorhydrat (65,2 g; 0,2 Mol) bei 20°C unter Rühren gegeben. Das Gemisch wird auf 115-120°C erwärmt und bei dieser Temperatur so lange gerührt, bis das feste Produkt in Lösung gegangen ist. Etwas Salzsäure wird bei dieser Temperatur ausgeschieden. Bei erhöhter Temperatur nimmt die Salzsäure-Entwicklung deutlich zu. Bei 180°C wird das Reaktionsgemisch als blass graues Produkt fest. Nach Abkühlung dieser Reaktionsmasse unterhalb 80°C wird kaltes Wasser zugegeben, worauf sich das Produkt in eine blass graue Suspension rasch umwandelt. Der Niederschlag wird dann abfiltriert, jedoch nicht gewaschen. Die Ausbeute ist quantitativ.

<u>Beispiel 3:</u> <u>3,3′-Dichlor-4,4′-diaminodiphenyl-5,5′-disulfonsäure ausgehend von 3,3′-Dichlorbenzidin-Dichlorhydrat in Lösung</u>

Zu 97,5 %igem Schwefelsäure (100 g) werden zuerst 25-30 % Oleum (160,4 g) dann getrocknetes 3,3′-Dichlorbenzidin-Dichlorhydrat-Pulver (65,2 g) gegeben. Das Gemisch wird auf 160°C erwärmt, bei dieser Temperatur während 30 Minuten gerührt und dann bis 50°C hinabgekühlt, wobei kein festes Produkt ausfällt. Das Gemisch wird in Eiswasser (1 Liter) langsam eingerührt, und das erhaltene Produkt wird abfiltriert. Nach dessen Reinigung beträgt die Ausbeute 85 %.

Das gemäss Beispiele 1 bis 3 erhaltene Rohprodukt kann nötigenfalls durch fraktionierte Ausfällung gereinigt werden. Das rohe Produkt wird in Wasser suspendiert, worauf 20%-iges wässriges Natriumhydroxid langsam zugegeben wird. Dabei bildet sich zuerst über eine dicke Phase (bei tiefem pH-Wert) eine Lösung bei pH 4-6, sofern die Produkt-Konzentration nicht zu hoch ist (etwa 4,5 Liter Lösung pro Mol Produkt bei pH 4-6 und 20°C Temperatur). Nach Zugabe von Tierkohle und Abfiltrieren über ein Filtrationshilfsmittel wird das erhaltene dunkel gefärbte Filtrat mit 5%-iger wässriger Salzsäure behandelt. Das über pH 3 ausgefallene Produkt wird abfiltriert und weggeworfen; das unter pH 3 (bis 0,5 hinab) ausgefallene Produkt wird dagegen abfiltriert und mehrmals obiger alkalischsaurer Behandlung unterworfen,

wobei jedesmal das Produkt bei tiefem pH-Wert und letzteres unter pH 0,5 isoliert wird. Reines Produkt kann durch Hochdruckflüssigkeits-Chromatographie über eine Phasenumkehr-Kolonne mit einer Reinheit von etwa 98 % nach bereits zwei aufeinanderfolgenden alkalisch-sauren Behandlungen gewonnen werden. Das Rohprodukt ist 91 % rein, wobei die Hauptverunreinigung (8 %) aus monosulfoniertem Produkt besteht. Schmelzpunkt des erhaltenen Produktes: > 300 ° C.

| Verbrennungsanalyse: (in %) | C | H | Cl | N | S | $H_2O$ |
|---|---|---|---|---|---|---|
| Für $C_{12}H_{10}Cl_2N_2O_6S_2.H_2O$ (Mol.Gew.: 431) | | | | | | |
| berechnet: | 33.4 | 2.8 | 16.5 | 6.5 | 14.9 | 4.2 |
| gefunden: | 34.8 | 2.8 | 16.5 | 6.6 | 14.9 | 3.5 |

IR-Absorption (Nujol-Anreibung):

$\lambda$max (in $cm^{-1}$): 3100 (b), 2700 (b), 2590 (st), 1600 (b), 1575 (m), 1530 (st), 1495 (m), 1365 (m-st), 1300 (s), 1265 (st), 1240 (st), 1220 (st), 1200 (st), 1120 (st,b), 1075 (s), 1065 (m), 1035 (st), 880 (st), 855 (s), 815 (st), 800 (m-st), 710 (m-st), 645 (st).
[b = breit; st = stark; m = mittel, s = schwach].

Titration einer alkalischen Lösung mit einer Standard-Säure zeigt die Anwesenheit von zwei monobasischen sauren Gruppen an.

Das Mono-Natriumsalz der Disulfonsäure kann aus einer NaOH-Lösung durch Ansäuern bis pH 1-3 erhalten werden. Daraus kann die freie Disulfonsäure mit Hilfe konzentrierter Salzsäure (> 20 %) gewonnen werden.

Beispiel 4: 3,3′-Dichlor-4,4′-diaminodiphenyl-5-sulfonsäure aus 3,3′-Dichlorbenzidin

3,3′-Dichlorbenzidin als freie Base (25,3 g; 0,1 Mol) und als getrocknetes feines Pulver wird zu 25-30 %igem Oleum (30 g; ca. 0,1 Mol $SO_3$) gegeben, und das Gemisch wird so lange mechanisch gerührt, bis das Amin durch das Oleum vollständig benetzt wird. Das dicke feste Produkt wird unter gelegentlichem Rühren auf 140 ° C erwärmt und bei dieser Temperatur während 30 Minuten gehalten, dann unterhalb 80 ° C abgekühlt. Wasser wird zugegeben, und das entstandene feste Produkt wird zu einer grauen Paste gemahlen. Diese Paste wird filtriert, und der Rückstand (ohne Waschen) wird dann wiederum in Wasser suspendiert, worauf so viel Natronlauge (als 20%-ige Lösung) zugegeben wird, bis der pH des Gemisches einen konstanten Wert von 12 aufzeigt. Unreagiertes 3,3′-Dichlorbenzidin wird abfiltriert (ca. 40 % Rückgewinnung), und das Filtrat wird mit verdünnter Salzsäure sorgfältig angesäuert (pH 1,5). Das rohe monosulfonierte Produkt wird durch Filtration isoliert, mit Wasser gewaschen und getrocknet. Ausbeute: 10,6 g (50 % der Theorie).

Beispiel 5: 3,3′-Dichlor-4,4′-diaminodiphenyl-5-sulfonsäure ausgehend von 3,3′-Dichlorbenzidin-Dichlorhydrat

Das 3,3′-Dichlorbenzidin-Dichlorhydrat wird zuerst bei 70 ° C während 16 Stunden getrocknet. Zu 25-30 %igem Oleum (60 g; ca. 0,206 Mol $SO_3$) wird getrocknetes 3,3′-Dichlorbenzidin-Dichlorhydrat (65,2 g; 0,2 Mol) gegeben. Die erhaltene dicke Suspension wird langsam erwärmt, es entsteht jedoch keine Lösung. Bei 155-160 ° C wird das Reaktionsgemisch fest und hart. Nach Abkühlung der Reaktionsmasse unterhalb 80 ° C wird dem Gemisch Wasser zugegeben, und die erhaltene dicke Suspension wird zu einer gleichmässigen Paste gemahlen. Natriumhydroxid als 20%-ige wässrige Lösung wird zugegeben, bis der pH der Suspension einen konstanten Wert von 11 aufzeigt. Unreagiertes 3,3′-Dichlorbenzidin (38 %) wird durch Filtration zurückgewonnen. Das Filtrat wird angesäuert (pH 2,5), und das resultierende monosulfonierte Produkt wird filtriert, mit eiskaltem Wasser gewaschen und getrocknet. Ausbeute: 26,4 g (49 % der Theorie).

Beispiel 6: 3,3′-Dichlor-4,4′-diaminodiphenyl-5-sulfonsäure ausgehend von 3,3′-Dichlorbenzidin-Dichlorhydrat in Lösung

Zu 97,5 %igem Schwefelsäure (100 g) werden zuerst 25-30 %-iges Oleum (100,4 g) dann getrocknetes gepulvertes 3,3′-Dichlorbenzidin-Dichlorhydrat (65,2 g) gegeben. Das Gemisch wird erwärmt und ist bei

100 ° C homogen. Schäumen hört bei 160 ° C auf, und das erhaltene Gemisch wid bei 160 ° C während 15 Minuten gerührt, dann abgekühlt, wobei unterhalb 60 ° C ein Produkt ausfällt. Das Gemisch wird in Eiswasser eingerührt, und das erhaltene Produkt wird abfiltriert. Ausbeute (nach Reinigung): ca. 50 %, bezogen auf das eingesetzte 3,3′-Dichlorbenzidin.

Das rohe, gemäss Beispiele 4, 5 oder 6 erhaltene Produkt kann nötigenfalls wie folgt gereinigt werden:

Das Rohprodukt wird in Wasser suspendiert, worauf NaOH zugegeben wird, bis der pH der Suspension 12 beträgt. Unreagiertes 3,3′-Dichlorbenzidin wird abfiltriert, und das erhaltene Filtrat wird mit Salzsäure bis auf pH 2,0 angesäuert. Die Monosulfonsäure wird abfiltriert und mehrmals einer alkalisch-sauren Ausfällung unterworfen. Bei pH-Werten unter 2,0 werden zunehmende Mengen Disulfonsäure erhalten.
Das Produkt kann mit Hochdruckflüssigkeits-Chromatographie über eine Phasenumkehr-Kolonne gereinigt werden und weist bereits nach 3 bis 4 aufeinanderfolgenden alkalisch-sauren Behandlungen eine Reinheit von 94 % auf.

| Verbrennungsanalyse: (in %) | C | H | Cl | N | S | $H_2O$ |
|---|---|---|---|---|---|---|
| Für $C_{12}H_{10}Cl_2N_2O_3S.H_2O$ | | | | | | |
| berechnet: | 41.04 | 3.44 | 20.19 | 7.98 | 9.13 | 5.13 |
| gefunden: | 40.6 | 3.34 | 19.1 | 7.61 | 9.1 | 5.3 |

IR-Absorption (Nujol-Anreibung):

λmax (in $cm^{-1}$): 3470 (st), 3370 (st), 2620 (b), 1620 (st) 1605 (st), 1300 (m), 1280 (s), 1215 (st), 1175 (st), 1100 (m-st), 1070 (s), 1030 (st), 895 (s), 870 (m), 830 (s), 820 (m), 800 (s), 735 (m), 695 (m), 640 (st).

Titration einer alkalischen Lösung mit einer Standard-Säure zeigt die Anwesenheit einer monobasischen sauren Gruppe an.

Beispiel 7: (Zur Illustration der Tetrazotierung der 3,3′-Dichlor-4,4′-diaminodiphenyl-5,5′-disulfonsäure und Kupplung mit Acetoacet-m-xylidid):

3,3′-Dichlor-4,4′-diaminodiphenyl-5,5′-disulfonsäure (41,3 g; 0,1 Mol) wird in 250 ml Wasser suspendiert und mit einer 20%igen wässrigen Natriumhydroxyid-Lösung bis pH 11,0 versetzt. Natriumnitrit (13,8 g; 0,2 Mol) in 50 ml Wasser wird zugegeben, und die erhaltene Mischung wird innerhalb von 5 Minuten in ein Gemisch aus 50 g konzentrierter Salzsäure und 1 Liter Eiswasser eingetragen, wobei sich die tief rotgefärbte Mischung rasch in eine ziegelrote Suspension des Tetrazoniumsalzes verwandelt. Ueberschüssige salpertrige Säure wird mit Hilfe einer Sulfaminsäure-Lösung kurz vor der Kupplung zersetzt.

Acetoacet-m-xylidid (41,0 g; 0,2 Mol) wird in einer wässrigen NaOH-Lösung (9 g in 500 ml Wasser) gelöst und dann durch Eintragen in 80%ige Eisessig (16 g) und Wasser (1 Liter) ausgefällt. Der pH-Wert wird auf 5,0 und die Temperatur auf 10 ° C eingestellt.

Die Tetrazoniumsalz-Suspension wird innerhalb von 45 Minuten zugegeben, wobei der pH durch gleichzeitige Zugabe einer 5%igen wässrigen NaOH-Lösung bei 5.0 konstant gehalten wird. Die Temperatur wird zwischen 10-20 ° C durch Eis-Zugabe (falls nötig) gehalten. Der Tetrazoniumsalz-Ueberschuss wird mit Hilfe einer H-Säure-Lösung kontrolliert. Nach Beendigung der Reaktion wird der pH auf 8 eingestellt, und die erhaltene Pigmentsuspension (enthaltend 1,7 % Farbstoff) wird gemäss Beispiele 8 und 9 weiter verwendet.

Beispiel 8: (Zur Illustration der Verwendung der gemäss Beispiel 7 erhaltenen Farbstoff-Suspension als Zusatz im Pigment Gelb C.I. Nr. 13):

1 Liter einer wässrigen Suspension des Pigments Gelb C.I. Nr. 13 (durch Tetrazotierung von 3,3′-Dichlorbenzidin in wässriger Lösung bei pH 4,5 hergestellt) enthaltend 8 % Pigmentfestanteil wird mit der nach Beispiel 7 hergestellten Suspension (118 g) versetzt, so dass ein Pigment mit 2,5 % Farbstoff erhalten wird. Dieses Produkt wird resiniert und auf übliche Art und Weise (z.B. gemäss Beispiel 4 der GB-PS Nr. 1356253) nachbehandelt. Das erhaltene Pigmentpulver wird in einen Oel-Firnis eingearbeitet. Die erhaltene Tinte-Ausfärbung weist eine verbesserte Farbstärke und Transparenz im Vergleich zu einer entsprechenden Ausfärbung, welche mit einem unbehandelten Pigment Gelb C.I. Nr. 13 (ohne Farbstoff-Zusatz) erhalten wurde, auf.

Beispiel 9: (Zur Illustration der Umwandlung der gemäss Beispiel 7 erhaltenen Farbstoff-Suspension in ein Metallsalz-Pigment):

1 Liter der Suspension gemäss Beispiel 7 [enthaltend etwa 17 g (0,02 Mol) Farbstoff] wird mit $CaCl_2 \cdot 2H_2O$ (6 g; 0,04 Mol) in Wasser (100 ml) versetzt, und die erhaltene Mischung wird auf 95 °C erwärmt, während 30 Minuten bei dieser Temperatur nachgerührt, abfiltriert, gewaschen, getrocknet und gemahlen. In Tinten, Anstrichfarben und Kunststoffe eingearbeitet weist das erhaltene Pigment nicht-blutende Eigenschaften auf.

Beispiel 10: (Zur Illustration der Tetrazotierung der 3,3′-Dichlor-4,4′-diaminodiphenyl-5-sulfonsäure und Kupplung mit Acetoacet-m-xylidid):

3,3′-Dichlor-4,4′-diaminodiphenyl-5-sulfonsäure (33,3 g; 0,1 Mol) wird in Wasser (250 ml) suspendiert und mit einer 20%igen wässrigen NaOH-Lösung bis pH 11,0 versetzt. Natriumnitrit (13,8 g; 0,2 Mol) in Wasser (50 ml) wird zugegeben, und das erhaltene Gemisch wird zuerst rasch, dann langsam, einer Mischung von 50 g konzentrierter Salzsäure und 1 Liter Wasser zugegeben. Zur Zersetzung überschüssiger salpetriger Säure wird Sulfaminsäure, als wässrige Lösung, kurz vor der Kupplung zugegeben.

Acetoacet-m-xylidid (41,0 g; 0,2 Mol) wird in einer Lösung von 9 g NaOH und 500 ml Wasser gelöst und durch Eintragen in 80%ige Eisessig (16 g) und Wasser (1 Liter) ausgefällt. Der pH-Wert wird auf 5,0 und die Temperatur auf 10 °C eingestellt.

Die Tetrazoniumsalz-Suspension wird innerhalb von 45 Minuten bei konstantem pH-Wert (5,0) unter gleichzeitiger Zugabe einer 5%igen wässrigen NaOH-Lösung zugegeben, wobei die Temperatur zwischen 10-20 °C durch Eis-Zugabe, falls nötig, gehalten wird. Ueberschüssiges Tetrazoniumsalz wird durch ständige Kontrolle mit H-Säure auf einen Minimalwert gehalten. Nach Beendigung der Reaktion wird der pH auf 7,0 gestellt. Die erhaltene Suspension (enthaltend 1,5 % Farbstoff) wird gemäss Beispiele 11 und 12 weiter verwendet.

Beispiel 11: (Zur Illustration der Verwendung der gemäss Beispiel 10 erhaltenen Farbstoff-Suspension als Zusatz in Pigment Gelb C.I. Nr. 13):

Das gleiche Verfahren wie im Beispiel 8 wird wiederholt, wobei aber anstelle der gemäss Beispiel 7 erhaltenen Suspension 133 g der gemäss Beispiel 10 erhaltenen Suspension verwendet werden.

Die mit dem so behandelten Pigment erhaltene Tinte (siehe Beispiel 8) weist eine verbesserte Farbstärke und Transparenz im Vergleich zur Tinte auf, die mit einem unbehandelten Pigment (ohne Farbstoff-Zusatz) hergestellt wurde.

Beispiel 12: (Zur Illustration der Umwandlung der Farbstoff-Suspension gemäss Beispiel 10 in ein Metallsalz-Pigment):

1 Liter der gemäss Beispiel 10 erhaltenen Suspension [enthaltend etwa 15,3 g (0,02 Mol) Farbstoff] wird mit $CaCl_2 \cdot 2H_2O$ (3 g, 0,02 Mol) in 50 ml Wasser versetzt. Das erhaltene Gemisch wird auf 95 °C erwärmt, während 30 Minuten bei dieser Temperatur gerührt, abfiltriert, gewaschen, getrocknet und gemahlen.

In Tinten, Anstrichfarben und Kunststoffe eingearbeitet weist dieses Pigment nicht-blutende Eigenschaften auf.

## Patentansprüche

**1.** Verbindungen der Formeln I oder II

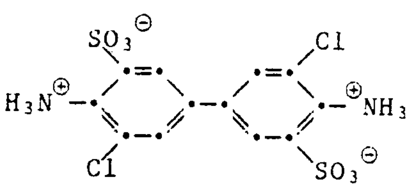 (I), 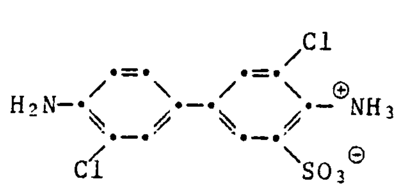 (II),

sowie deren Salze und Hydrate.

2. Verfahren zur Herstellung einer Verbindung der Formeln I oder II gemäss Anspruch 1, wonach man 3,3′-Dichlorbenzidin oder dessen Dichlorhydrat mit einer entsprechenden stöchiometrischen Menge Oleum bei einer Temperatur zwischen 90 ° C und 180 ° C reagieren lässt.

3. Verfahren gemäss Anspruch 2, wobei die Konzentration des Oleums zwischen 20 und 30 % liegt.

4. Verfahren gemäss Anspruch 2, wobei die Reaktionstemperatur zwischen 100 und 160 ° C liegt.

5. Verfahren gemäss Anspruch 2, wobei 3,3′-Dichlorbenzidin-Dichlorhydrat und als Lösungsmittel konzentrierte Schwefelsäure verwendet werden.

6. Verwendung der Verbindungen der Formeln I oder II gemäss Anspruch 1 als Zwischenprodukte für Farbstoffzusätze in Pigmentzusammensetzungen.

7. Verwendung der Verbindungen der Formeln I oder II gemäss Anspruch 1 als Zwischenprodukte für Metall enthaltende Pigmente.

## Claims

1. A compound of formula I or II

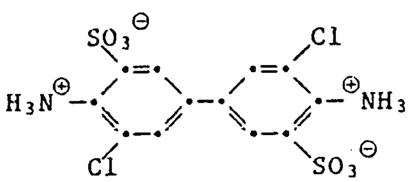 (I) 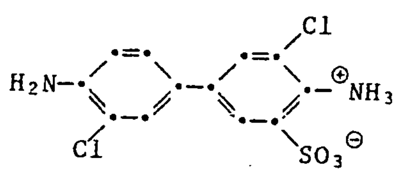 (II)

and its salts and hydrates.

2. A process for the preparation of a compound of formula I or II according to claim 1, which comprises reacting 3,3'-dichlorobenzidine or its dihydrochloride with an appropriate stoichiometric amount of oleum at a temperature between 90 ° C and 180 ° C.

3. A process according to claim 2, wherein the concentration of the oleum is between 20 and 30 %.

4. A process according to claim 2, wherein the reaction temperature is between 100 and 160 ° C.

5. A process according to claim 2, wherein 3,3'-dichlorobenizidine dihydrochloride and, as solvent, concentrated sulphuric acid are used.

6. Use of compounds of formula I or II according to claim 1 as intermediates for dyestuff additives in pigment compositions.

7. Use of compounds of formula I or II according to claim 1 as intermediates for metal-containing pigments.

## Revendications

1. Composés répondant à l'une des formules I et II :

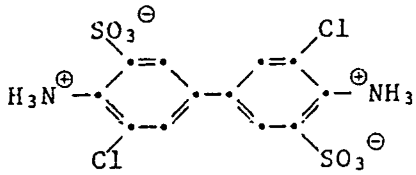 (I), 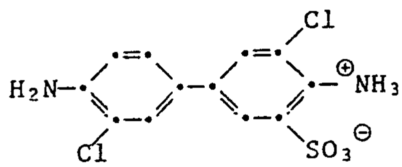 (II),

ainsi que leurs sels et leurs hydrates.

**2.** Procédé pour préparer un composé de formule I ou II selon la revendication 1, procédé selon lequel on fait réagir la 3,3'-dichloro-benzidine, ou son dichlorhydrate, avec une quantité stoechiométrique correspondante d'oléum, à une température comprise entre 90 et 180 °C.

**3.** Procédé selon la revendication 2 selon lequel la concentration de l'oléum est comprise entre 20 et 30 %.

**4.** Procédé selon la revendication 2 selon lequel la température réactionnelle est comprise entre 100 et 160 °C.

**5.** Procédé selon la revendication 2 selon lequel on utilise le dichlorhydrate de 3,3'-dichloro-benzidine et, comme solvant, de l'acide sulfurique concentré.

**6.** Application des composés de formules I et II selon la revendication 1 comme produits intermédiaires pour des additifs colorants dans des compositions pigmentaires.

**7.** Application des composés de formules I et II selon la revendication 1 comme produits intermédiaires pour des pigments contenant un métal.